Europäisches Patentamt

European Patent Office

Office européen des brevets

(11) Veröffentlichungsnummer: **0 174 543**
**A1**

# (12) EUROPÄISCHE PATENTANMELDUNG

(21) Anmeldenummer: 85110685.6

(22) Anmeldetag: 26.08.85

(51) Int. Cl.⁴: **C 07 C 97/24**

(30) Priorität: 06.09.84 DE 3433074

(43) Veröffentlichungstag der Anmeldung:
19.03.86 Patentblatt 86/12

(84) Benannte Vertragsstaaten:
CH DE FR GB LI

(71) Anmelder: BAYER AG
Konzernverwaltung RP Patentabteilung
D-5090 Leverkusen 1 Bayerwerk(DE)

(72) Erfinder: Ockelmann, Dieter, Dr.
Gerstenkamp 10
D-5000 Köln 80(DE)

(72) Erfinder: Schmitz, Reinold, Dr.
Im Kerberich 36
D-5068 Odenthal(DE)

(72) Erfinder: Schneider, Jürgen, Dr.
Am Theiensiefen 9
D-5068 Odenthal(DE)

(72) Erfinder: Maertens, Dieter, Dr.
Dudweiler Strasse 2c
D-5090 Leverkusen 1(DE)

(54) Verfahren zur Erzeugung von Aminoanthrachinonen mit verbesserten verarbeitungstechnischen Eigenschaften.

(57) Verfahren zur Erzeugung von Aminoanthrachinonen mit verbesserten verarbeitungstechnischen Eigenschaften gemäß dem man die bei der Reduktion von Nitroanthrachinonen mit wäßrigen Sulfid-Lösungen erhaltenen Reaktionsgemische einer thermischen Behandlung unterwirft.

EP 0 174 543 A1

0174543

– 1–

BAYER AKTIENGESELLSCHAFT        5090 Leverkusen, Bayerwerk

Konzernverwaltung RP        B/by-c
Patentabteilung


Verfahren zur Erzeugung von Aminoanthrachinonen mit
verbesserten verarbeitungstechnischen Eigenschaften

Die Erfindung betrifft ein Verfahren zur Erzeugung von
Aminoanthrachinonen mit verbesserten verarbeitungstechnischen Eigenschaften.

Es ist bekannt, Aminoanthrachinone durch Reduktion von
Nitroanthrachinonen mit Sulfiden herzustellen (siehe
z.B. die GB-PS 1 346 889 und US-PS 4,407,756) Dieses Reduktionsverfahren zeichnet sich durch seine einfache Durchführbarkeit und auch dadurch aus, daß es die Aminoanthrachinone sowohl in hohen Ausbeuten als auch in
guter Qualität liefert. Nachteilig an dem Verfahren
ist jedoch, daß die Aminoanthrachinone bei der Reduktion in einer schlecht filtrierbaren Form anfallen
und daß deshalb die Isolierung der Aminoanthrachinone
aus den bei der Reduktion anfallenden Reaktionsgemischen erhebliche Schwierigkeiten bereitet.

Zur Vermeidung dieser Schwierigkeiten wird in der
US-PS 4,407,756 vorgeschlagen, die Reduktion nicht
diskontinuierlich sondern kontinuierlich in der Weise
durchzuführen, daß man Nitroanthrachinone und Alkali-

Le A 23 371

sulfide einer auf einer bestimmten Temperatur gehaltenen Reaktionszone zuführt und das Reaktionsgemisch kontinuierlich abzieht. Diese Arbeitsweise führt zwar zu besser filtrierbaren Aminoanthrachinonen. Die so erhaltenen Aminoanthrachinone haben jedoch den Nachteil, daß sie erhebliche Mengen an anorganischen Bestandteilen enthalten, die sich nur unter erheblichem Aufwand durch Waschen entfernen lassen.

Mit dem in der DE-OS 3 306 532 beschriebenen Verfahren zur Reinigung von 1-Aminoanthrachinon wird eine Verbesserung der Filtrierbarkeit des im Reduktionsgemisch vorliegenden 1-Aminoanthrachinons weder bezweckt noch erreicht.

Es wurde nun gefunden, daß sich die nach der Reduktion von Nitroanthrachinonen mit Sulfid erhaltenen Aminoanthrachinone in eine ausgezeichnet abtrennbare Form überführen lassen, wenn man das nach der Reduktion vorliegende Reaktionsgemisch einer thermischen Behandlung unterwirft.

Die Erfindung betrifft daher ein Verfahren zur Erzeugung von Aminoanthrachinonen mit verbesserten verarbeitungstechnischen Eigenschaften (verbesserter Filtrier- und Auswaschbarkeit), das dadurch gekennzeichnet ist, daß man die bei der Reduktion von Nitroanthrachinonen mit wäßrigen Sulfid-Lösungen erhaltenen Reaktionsgemische einer thermischen Behandlung unterwirft.

Le A 23 371

Die thermische Behandlung besteht in einem Erwärmen
des Reaktionsgemisches auf Temperaturen von 120 bis
200°C, vorzugsweise 130 bis 170°C.

Das Erwärmen des Reaktionsgemisches wird in geschlossenen
Gefäßen, d.h. unter dem sich bei der gewählten Behandlungstemperatur im Gefäß einstellenden Dampfdruck,
d.h. bei Drucken von 2 bis 16 bar, vorzugsweise 2,5 bis
8 bar, vorgenommen.

Bei der erfindungsgemäßen thermischen Nachbehandlung
findet offensichtlich eine Rekristallisation der im
Reaktionsgemisch zunächst in feinkristalliner und
teilweise agglomerierter Form vorliegenden Aminoanthrachinone statt. Bei dieser Rekristallisation
findet jedoch nicht nur eine Umwandlung der feinkristallinen, schlecht filtrierbaren Aminoanthrachinone
in gröberkristalline, besser filtrier- und auswaschbare
Aminoanthrachinone statt, sondern auch eine Reinigung der
Aminoanthrachinone, denn bei der Rekristallisation werden die in den feinkristallinen, agglomerierten Aminoanthrachinonen eingeschlossenen Reste an Nitroanthrachinonen und Hydroxylamino-anthrachinonen freigesetzt
und zu Aminoanthrachinonen reduziert.

Der Verlauf der thermischen Nachbehandlung läßt sich
analytisch und optisch verfolgen. Die Behandlung
wird solange durchgeführt, bis der Restgehalt an Nitro-
und Hydroxylamino-anthrachinonen im Reaktionsgemisch
auf unter 0,3 Gew.-%, bezogen auf das Gewicht an

Le A 23 371

Aminoanthrachinon, abgesunken ist und sich die ursprünglich im Reaktionsgemisch vorliegenden feinen, zum Teil agglomerierten Kristalle in gröbere Kristalle umgewandelt haben. In Abhängigkeit von der Durchmischung und Größe des Reaktionsansatzes dauert die thermische Nachbehandlung 30 Minuten bis 16 Stunden, vorzugsweise 1 bis 6 Stunden.

Nach der erfindungsgemäßen thermischen Nachbehandlung werden Aminoanthrachinone, z.B. 1-Aminoanthrachinon, mit einem wesentlich geringeren Gehalt an anorganischen Verbindungen erhalten, als nach dem in der DE-OS 3 306 532 beschriebenen Reinigungsverfahren. Gemäß dem in der DE-OS 3 306 532 beschriebenen Verfahren wird ein 1-Aminoanthrachinon erhalten, dessen Gehalt an anorganischen Verbindungen unter 4 % liegt; nach der erfindungsgemäßen Nachbehandlung liegt der Gehalt des 1-Aminoanthrachinons an anorganischen Salzen unter 0,5 Gew.-%. Ein weiterer Vorteil des erfindungsgemäßen thermischen Nachbehandlungsverfahrens besteht darin, daß der feuchte Filterkuchen des abfiltrierten Aminoanthrachinons einen wesentlich höheren Feststoffgehalt, etwa 70 Gew.-%, aufweist als die Filterkuchen der nach den bekannten Verfahren erhaltenen Aminoanthrachinone, deren Gehalt an Feststoffen nur etwa 30 Gew.-% beträgt.

Die erfindungsgemäße thermische Behandlung eignet sich grundsätzlich für die Nachbehandlung aller Reaktionsgemische wie sie bei der Reduktion von Nitroanthrachinonen, z.B. Mononitroanthrachinonen, wie 1-Nitroanthrachinon, oder Dinitroanthrachinonen, wie 1,5- oder 1,8-Dinitroanthrachinon, mit wäßrigen

Le A 23 371

Sulfid-Lösungen anfallen. Die Reduktionen können kontinuierlich oder diskontinuierlich ausgeführt worden sein. Die Reduktionen werden vorzugsweise bei Temperaturen von 50 bis 100°C, vorgenommen. Je Gewichtsteil Anthrachinon werden vorzugsweise 3 bis 15 Gewichtsteile Wasser und je Mol Nitrogruppe 1,6 bis 3,0 Mol Sulfid angewendet. Als Sulfide kommen vor allem die neutralen oder sauren Ammonium-, Alkali- oder Erdalkalisalze von Schwefelwasserstoffen in Frage. Zur Verbesserung der Rekristallisations- und Reduktionswirkung können den wäßrigen Gemischen kleine Mengen oberflächenaktiver Mittel, z.B. Ether oder Ester von Polyethylenglykolen zugesetzt werden.

Bei den Konzentrationsangaben in den folgenden Beispielen handelt es sich, sofern nichts anderes angegeben ist, um Gewichtsprozente.

Le A 23 371

### Beispiel 1

In die auf 90°C erwärmte Lösung aus 0,2 g eines polyethoxylierten Fettsäurealkohols, 100 ml 28 %iger Natriumhydrogensulfidlösung und 33 ml 50 %iger Natronlauge werden innerhalb von 30 Minuten 65 g 1-Nitroanthrachinon (97,4 %iges Produkt) eingetragen. Zur Beendigung der Reduktion wird das Reaktionsgemisch 30 Minuten bei 90°C gerührt.

Die eine Hälfte des Ansatzes wird anschließend noch 4 Stunden auf 90 bis 95°C erwärmt. Der Niederschlag wird heiß abfiltriert, mit heißem Wasser neutral gewaschen und bei 100°C getrocknet. Man erhält auf diese Weise 28,7 g eines feinkristallinen 95,3 %igen 1-Aminoanthrachinons (Gehalt an Nitro- und Hydroxylaminoanthrachinon: 1,1 Gew.-%). Der Feststoffgehalt des feuchten, thixotropen Filterkuchens beträgt etwa 30 Gew.-%.

Die andere Hälfte des Ansatzes wird in einem Autoklaven 4 Stunden auf 150°C erhitzt und nach dem Abkühlen auf 90°C, wie vorstehend beschrieben, aufgearbeitet. Es werden auf diese Weise 28,2 g eines grobkristallinen 97,4 %igen 1-Amino-anthrachinons erhalten (Gehalt an Nitro- und Hydroxylamino-anthrachinon: 0,1 Gew.-%). Der Feststoffgehalt des feuchten Filterkuchens beträgt etwa 70 Gew.-%.

Le A 23 371

Beispiel 2

Bei Raumtemperatur werden 35 g 1-Nitroanthrachinon (97,4 %iges Produkt), 250 ml Wasser und 66 ml 28 %ige Natriumhydrogensulfid-Lösung miteinander verrührt. Die Mischung wird 1 Stunde auf 90 bis 95°C erwärmt und anschließend im Autoklaven 2 Stunden auf 140°C erhitzt.

Die Aufarbeitung des Reaktionsgemisches wird wie in Beispiel 1 beschrieben vorgenommen.

Es werden 30,2 g eines grobkristallinen 97,6 %igen 1-Amino-anthrachinons erhalten (Gehalt an Nitro- und Hydroxylamino-anthrachinon: 0,2 Gew.-%). Der Feststoffgehalt des feuchten Filterkuchens beträgt etwa 64 Gew.-%.

Beispiel 3

62,5 g 1-Nitro-anthrachinon (98,25 %iges Produkt) werden bei Raumtemperatur in der Lösung von 0,4 g eines polyethoxylierten Fettalkohols in 257 ml Wasser suspendiert. Die Suspension wird unter Rühren mit 102 ml einer 27,5 %igen Natriumhydrogensulfid-Lösung versetzt. Die Mischung wird 30 Minuten bei 90 bis 95° gerührt. Anschließend wird das Reaktionsgemisch im Autoklaven 3 Stunden auf 150°C erhitzt.

Die Aufarbeitung des Reaktionsgemisches wird wie in Beispiel 1 beschrieben vorgenommen. Es werden 54,4 g eines grobkristallinen 98,9 %igen 1-Aminoanthrachinons erhalten (Gehalt an Nitro- und Hydroxylamino-anthrachinon: 0,1 Gew.-%). Der Feststoffgehalt des feuchten Filterkuchens beträgt etwa 68 Gew.-%.

Le A 23 371

Beispiel 4

Die Suspension von 125 g 1-Nitroanthrachinon (98,3 %iges Produkt) in 534 ml Wasser wird bei Raumtemperatur mit 205 ml einer 27,5 %igen Natriumhydrogensulfid-Lösung versetzt. Die Reaktionsmischung wird innerhalb von 45 Minuten auf 170°C erhitzt und 6 Stunden auf dieser Temperatur gehalten.

Das Reaktionsgemisch wird wie in Beispiel 1 beschrieben aufgearbeitet. Es werden 107,8 g eines grobkristallinen 98,9 %igen 1-Aminoanthrachinons erhalten (Gehalt an Nitro- und Hydroxylamino-anthrachinon: < 0,1 Gew.-%). Der Trockengehalt des feuchten Filterkuchens beträgt etwa 72 Gew.-%.

Beispiel 5

In die Lösung aus 465 ml Wasser, 0,5 g polyethoxyliertem Fettalkohol und 197 ml 28 %iger Natriumhydrogensulfid-Lösung werden bei 80°C 60 g 1,5-Dinitro-anthrachinon (97,85 %iges Produkt) unter Rühren so eingetragen, daß die Temperatur der Reaktionsmischung nicht über 100°C ansteigt. Das Reaktionsgemisch wird 30 Minuten bei 100°C nachgerührt und anschließend 4 Stunden auf 140°C erwärmt.

Die Aufarbeitung des Reaktionsgemisches wird wie in Beispiel 1 beschrieben vorgenommen. Es werden 47,4 g eines grobkristallinen 89,9 %igen 1,5-Diamino-Anthrachinons erhalten (Gehalt an Nitro- und Hydroxyl-amino-Derivaten: 0,25 Gew.-%). Der Trockengehalt des feuchten Filterkuchens beträgt etwa 50 Gew.-%.

Le A 23 371

Wird das Reaktionsgemisch nur 4 Stunden bei 100°C nachgerührt, so werden 47,3 g eines 95,5 %igen sehr feinkristallinen 1,5-Diamino-anthrachinons erhalten (Gehalt an Nitro- und Hydroxylamino-Derivaten: 3,5 Gew.-%). Der Trockengehalt des feuchten,thixotropen Filterkuchens beträgt etwa 25 Gew.-%.

Beispiel 6

Die Suspension von 66 g 1,5-Dinitro-anthrachinon (97,85 %iges Produkt) in der Lösung von 28 ml einer 2 %igen Lösung eines polyethoxylierten Fettsäurealkohols in 465 ml Wasser wird bei Raumtemperatur mit 197 ml einer 28 %igen Natriumhydrogensulfid-Lösung versetzt. Die Reaktionsmischung wird im Autoklaven innerhalb von 45 Minuten auf 170°C erhitzt und 4 Stunden auf dieser Temperatur gehalten.

Die Aufarbeitung des Reaktionsgemisches wird wie in Beispiel 1 beschrieben vorgenommen. Es werden 51,8 g eines grobkristallinen 99,25 %igen 1,5-Diaminoanthrachinons erhalten (Gehalt an Nitro- und Hydroxylaminoanthrachinon-Derivaten: 0,2 Gew.-%) Der Trockengehalt des feuchten Filterkuchens beträgt etwa 55 Gew.-%.

## Patentansprüche

1. Verfahren zur Erzeugung von Aminoanthrachinonen mit verbesserten verarbeitungstechnischen Eigenschaften, dadurch gekennzeichnet, daß man die bei der Reduktion von Nitroanthrachinonen mit wäßrigen Sulfid-Lösungen erhaltenen Reaktionsgemische einer thermischen Behandlung unterwirft.

2. Verfahren gemäß Anspruch 1, dadurch gekennzeichnet, daß die thermische Behandlung in einem Erhitzen des Reaktionsgemisches in einem geschlossenen Gefäß auf Temperaturen von 120 bis 200°C besteht.

3. Verfahren gemäß Anspruch 1, dadurch gekennzeichnet, daß die thermische Behandlung in einem Erhitzen des Reaktionsgemisches in einem geschlossenen Gefäß auf Temperaturen von 130 bis 170°C besteht.

4. Verfahren gemäß Ansprüchen 1 bis 3, dadurch gekennzeichnet, daß die thermische Behandlung solange vorgenommen wird, bis der Restgehalt an Nitro- und Hydroxylamino-anthrachinonen im Reaktionsgemisch auf unter 0,3 Gew.-%, bezogen auf das Gewicht an Aminoanthrachinonen, abgesunken ist und sich die ursprünglich im Reaktionsgemisch vorliegenden feinen, zum Teil agglomerierten Kristalle in gröbere Kristalle umgewandelt haben.

Le A 23 371

Europäisches Patentamt

**EUROPÄISCHER RECHERCHENBERICHT**

| EINSCHLÄGIGE DOKUMENTE | | | EP 85110685.6 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| D,Y | <u>US - A - 4 407 756</u> (FUKASAWA et al.) <br><br> * Ansprüche; Beispiele 1,2 * <br><br> -- | 1,2 | C 07 C 97/24 |
| Y | <u>DE - B2 - 2 410 310</u> (BAYER) <br><br> * Ansprüche 1,2; Beispiel 2 * <br><br> -- | 1,2 | |
| A | PATENT ABSTRACTS OF JAPAN, unexamined applications, Sektion C, Band 2, Nr. 85, 12. Juli 1978 <br><br> THE PATENT OFFICE JAPANESE GOVERNMENT <br> Seite 1246 C 78 <br><br> * Kokai-Nr. 53-44 550 (SUMITOMO) * <br><br> -- | 1-3 | |
| A | PATENT ABSTRACTS OF JAPAN, unexamined applications, Field C, Band 7, Nr. 225, 6. Oktober 1983 <br><br> THE PATENT OFFICE JAPANESE GOVERNMENT <br> Seite 3 C 189 <br><br> * Kokai-Nr. 58-118 547 (SUMITOMO) * <br><br> -- | 1 | RECHERCHIERTE SACHGEBIETE (Int Cl 4) <br><br> C 07 C 97/00 |
| A | <u>DE - A1 - 3 306 532</u> (SUMITOMO) <br><br> * Ansprüche 1,2,6; Beispiele 3,4 * <br><br> ---- | 1 | |

Der vorliegende Recherchenbericht wurde für alle Patentansprüche erstellt.

| Recherchenort | Abschlußdatum der Recherche | Prüfer |
|---|---|---|
| WIEN | 13-11-1985 | KÖRBER |